# EUROPEAN PATENT APPLICATION

(11) **EP 2 944 632 A1**
(43) Date of publication of application: **18.11.2015**
(21) Application number: 15163708.9
(22) Date of filing: 12.12.2008
(51) Int. Cl.: C07D 307/68, C07D 307/54, C07D 307/50

(54) **OXIDATION OF A MIXTURE OF 2-HYDROXYMETHYL-5-FURFURALDEHYDE (HMF) AND AN ESTER THEREOF TO 2,5-FURANDICARBOXYLIC ACID (FDCA)**

(30) Priority: 12.12.2007 US 996946 P; 14.12.2007 US 6012 P
(62) Divisional of application: 11190670.7
(71) Applicant: Archer Daniels Midland Company, Decatur, IL 62526 (US)
(72) Inventor: Sanborn, Alexandra, Lincoln, IL 62656 (US); Howard, Stephen, Sherman, IL 62684 (US)
(74) Representative: Von Kreisler Selting Werner - Partnerschaft von Patentanwälten und Rechtsanwälten mbB

(57) **Abstract**

A method for oxidizing a reaction mixture of 2-hydroxymethyl-5-furfuraldehyde (HMF) and an ester of 2-hydroxymethyl-5-furfuraldehyde (HMF) to 2,5-furandicarboxylic acid (FDCA) by the addition of cobalt acetate, manganese acetate, sodium bromide under pressure and heat to form FDCA.

## Description

### CROSS REFERENCE TO PROVISIONAL APPLICATION

This application is based upon and claims the benefit of priority from Provisional US Patent Application 61/006,012 (Attorney Docket No. 010253-0020) filed on December 14, 2007, and from Provisional US Patent Application 60/996,946 (Attorney Docket No. 010253-0021) filed on December 12, 2007, the entire contents of which are incorporated by reference herein.

### TECHNICAL FIELD

The present invention relates to a process for the synthesis and recovery of substantially pure HMF and derivatives thereof from hexose carbohydrate feedstocks such as fructose or high fructose corn syrup (HFCS). More particularly, HMF and its derivatives are synthesized, separated, and recovered via contact of the carbohydrate with strong acid cation exchange resins, such as a solid phase catalyst.

### BACKGROUND

A major product in the acid-catalyzed dehydration of fructose is 2-hydroxymethyl-5-furfuraldehyde, also known as hydroxymethylfurfural (HMF). The structure of HMF is shown below:

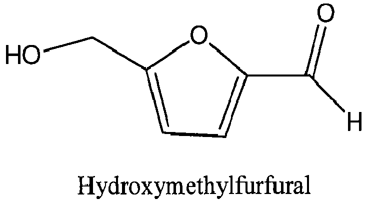

HMF represents one key intermediate substance readily accessible from renewable resources like carbohydrates and is a suitable starting source for the formation of various furan monomers which are used for the preparation of non-petroleum-derived polymeric materials. While not being bound by theory, it is generally believed that fructose is converted to HMF via an acyclic pathway, although evidence also exists for the conversion to HMF via cyclic fructofuransyl intermediate pathways. Regardless of the mechanism of HMF formation, the intermediate species formed during the reaction may in turn undergo further reactions such as condensation, rehydration, reversion and other rearrangements, resulting in a plethora of unwanted side products. Below is one proposed pathway for the conversion of fructose to HMF:

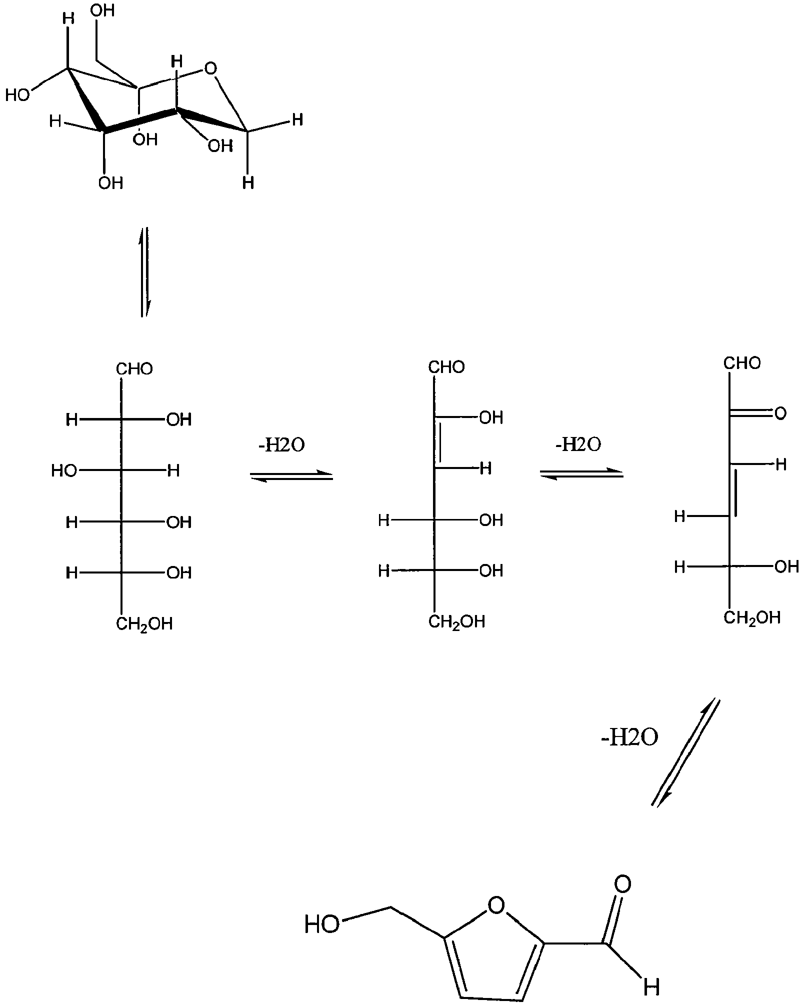

HMF and 2,5-disubstituted furanic derivatives have great potential in the field of intermediate chemicals from regrowing resources. Due to its various functionalities, it has been proposed that HMF could be utilized to produce a wide range of products such as polymers, solvents, surfactants, pharmaceuticals, and plant protection agents, and has been reported to have antibacterial and anticorrosive properties. HMF is also a key component, as either a starting material or intermediate, in the synthesis of a wide variety of compounds, such as furfuryl dialcohols, dialdehydes, esters, ethers, halides and carboxylic acids.

In addition, HMF has great potential as a biofuel, which are fuels derived from biomass and are considered promising alternatives to fossil fuels. HMF is also currently under investigation as a treatment for sickle cell anemia. In short, HMF is an important chemical compound and a method of synthesis on a large scale to produce HMF absent significant amounts of impurities, side products and remaining starting material has been sought for nearly a century.

HMF is a suitable starting source for the formation of various furan monomers used in the preparation of non-petroleum-derived polymeric materials. A furan is a 5-membered heterocyclic organic compound. HMF and 2,5-disubstituted furanic derivatives have great potential in the field of intermediate chemicals from growing resources. Due to its various functionalities, it has been proposed that HMF may be utilized to produce a wide range of products such as polymers, solvents, surfactants, pharmaceuticals, and plant protection agents, and HMF has been reported to have antibacterial and anticorrosive properties.

Although preparation of HMF has been known for many years, a method which provides HMF with good selectivity and in high yields has yet to be found. Complications arise from the rehydration of HMF, which yields by-products, such as, levulinic and formic acids. Another unwanted side reaction includes the polymerization of HMF and/or fructose resulting in humin polymers, which are solid waste products. Further complications may arise as a result of solvent selection. Water is easy to dispose of and dissolves fructose, but unfortunately, low selectivity and increased formation of polymers and humin increases under aqueous conditions.

Agricultural raw materials such as starch, cellulose, sucrose or inulin are inexpensive starting materials for the manufacture of hexoses, such as glucose and fructose. As shown above, these hexoses can in turn, be converted to HMF. The dehydration of sugars to produce HMF is well known. HMF was initially prepared in 1895 from levulose by Dull (Chem. Ztg., 19, 216) and from sucrose by Kiermayer (Chem. Ztg., 19, 1003). However, these initial syntheses were not practical methods for producing HMF due to low conversion of the starting material to product.

Commonly used catalysts for the preparation of HMF includes cheap inorganic acids such as H₂SO₄, H₃PO₄, and HCl. These acid catalysts are used in solution and are difficult to regenerate. In order to avoid the regeneration and disposal problems, solid sulfonic acid catalysts have been used. Unfortunately, the usefulness of solid acid resins is limited because of the formation of deactivating humin polymers on the surface of the resins.

The purification of HMF has also proved to be a troublesome operation. On long exposure to temperatures at which the desired product can be distilled, HMF and impurities associated with the synthetic mixture tend to form tarry degradation products. Because of this heat instability, a falling film vacuum still must be used. Even in such an apparatus, resinous solids form on the heating surface causing a stalling in the rotor and frequent shut down time making the operation inefficient. Prior work has been performed with distillation and the addition of a non-volatile solvent like PEG-600 to prevent the buildup of solid humin polymers (Cope, U.S. Patent No. 2,917,520). Unfortunately, the use of polyglycols leads to the formation of HMF-PEG ethers.

The prior art processes also fail to provide a method for producing HMF that can be performed economically. For example, Besemer et al Netherlands Organ. Appl. Sci. Res. Nutr. Food Res., describes the enzymatic synthesis of HMF esters. This process requires the use of expensive enzymes and therefore does not provide an economically feasible route to synthesizing HMF esters.

Garber et al., Canadian Patent 6 54240, describe the synthesis of the 2,5-tetrahydrofurandimethanol monoesters from HMF using excess amounts of anhydride and pyridine solvent. Reduction is performed using Raney Ni catalyst in diethyl ether. However the reference does not disclose the synthesis of HMF esters from fructose or using a carboxylic acid. Furthermore, the removal of Raney Ni catalyst is dangerous and the costs of disposing the catalyst may be burdensome.

The present disclosure, which is directed, in-part, to chromatographic processes for the synthesis and recovery of HMF from natural resources addresses and eliminates these problems and provides high purity products. In addition to HMF, studies have broadened to include the synthesis and purification of a variety of HMF derivatives. Derivatives of particular interest include the esters of HMF, and oxidized forms (2,5-diformylfuran, 2,5-furandicarboxylic acid and acid ester), and the reduced forms (furan-2,5-dimethanol and tetrahydrofuran diol) of HMF. The esters are more stable and can be readily separated, potentially making them even more useful than HMF itself.

### SUMMARY OF THE DISCLOSURE

In order to address the above mentioned problems, the disclosure provides a method of producing substantially pure HMF, HMF esters or HMF ethers from a carbohydrate source by contacting the carbohydrate source with a solid phase catalyst. In the present disclosure substantially pure means a purity of HMF of about 70% or greater, optionally about 80% or greater, or about 90% or greater.

The disclosure also provides a method of producing HMF esters from a carbohydrate source and organic acids. In one embodiment, a carbohydrate starting material is heated with an solvent in a column and continuously flowed through a solid phase catalyst in the presence of an organic acid to form a HMF ester. The solvent is removed by rotary evaporation to provide a substantially pure HMF ester. In another embodiment, a carbohydrate is heated with the organic acid and a solid catalyst in a solution to form an HMF ester. The resulting HMF ester may then be purified by filtration, evaporation, extraction, and distillation or any combination thereof.

In another embodiment, there is provided a method for oxidizing an HMF ester to 2,5-furandicarboxylic acid (FDCA) by combining the HMF ester with an organic acid, cobalt acetate, manganese acetate and sodium bromide under pressure and to obtain substantially pure FDCA after filtration and evaporation.

In another embodiment, there is provided a method of oxidizing a reaction mixture of HMF and HMF ester to FDCA by the addition of cobalt acetate, manganese acetate, and sodium bromide under pressure and heat and isolating FDCA following filtration and evaporation.

In an alternative embodiment, there is provided a method of reducing an HMF ester by the addition of an alcohol, such as ethanol, a reducing agent, under pressure, heat, filtration and evaporation.

Advantages of the methods as described herein are the high rate of conversion of carbohydrates into HMF-esters and derivatives. This results in a more stable form for HMF, and a lower cost in materials.

In another embodiment, there is provided a method for producing citrate esters from a citric acid source and an alcohol. Citric acid is esterified with an alcohol in the presence of a catalyst on a chromatography column to produce trialkyl citrate or mono- and di-esters of the citric acid. In an alternative embodiment, a fermentation broth containing primarily citric acid and residual microorganisms and fermentation side products is used to produce trialkyl citrate or mono- and di-esters of the citric acid. In another embodiment, the mono- and di-esters are recycled through the catalyst and column to generate the triester.

In yet an another embodiment, there is provided a method of preparing HMF via deacylation of an intermediate HMF ester. In one embodiment of this method, fructose is dehydrated in the presence of an organic acid and a catalyst, and separated via a chromatography column to produce the HMF ester. In an alternative embodiment, the HMF ester is deacylated with a solid phase catalyst in a chromatography column. Alternatively, the deacylation and separation of the HMF ester is performed using a metal alkoxide.

In another embodiment, there is provided a method for the synthesis of levulinic acid or levulinic ester by contacting a carbohydrate mixture with or without an organic acid present, with a solid phase catalyst under elevated temperature. HMF ethers and/or levulinate esters, which are more stable than HMF may be synthesized and purified by this process using an alcohol solvent. Advantages of the methods as described herein are the high rate of conversion of carbohydrates into substantially pure HMF, HMF esters and other HMF derivatives.

### BRIEF DESCRIPTION OF THE DRAWINGS

Fig. 1 illustrates a conventional autoclave reactor;
Fig. 2 illustrates the fraction of AcHMF conversion in a conventional method using an autoclave reactor;
Fig. 3 illustrates the fraction of AcHMF conversion according to an embodiment of the present application;
Fig. 4 illustrates a graph of the products using the pulse resin test according to an embodiment of the present application; and
Fig. 5 illustrates a chromatogram according to an embodiment of the present application.
Fig. 6 is a ¹H NMR analysis graph showing substantially pure 4-acetoxymethylfurfural (AcHMF).

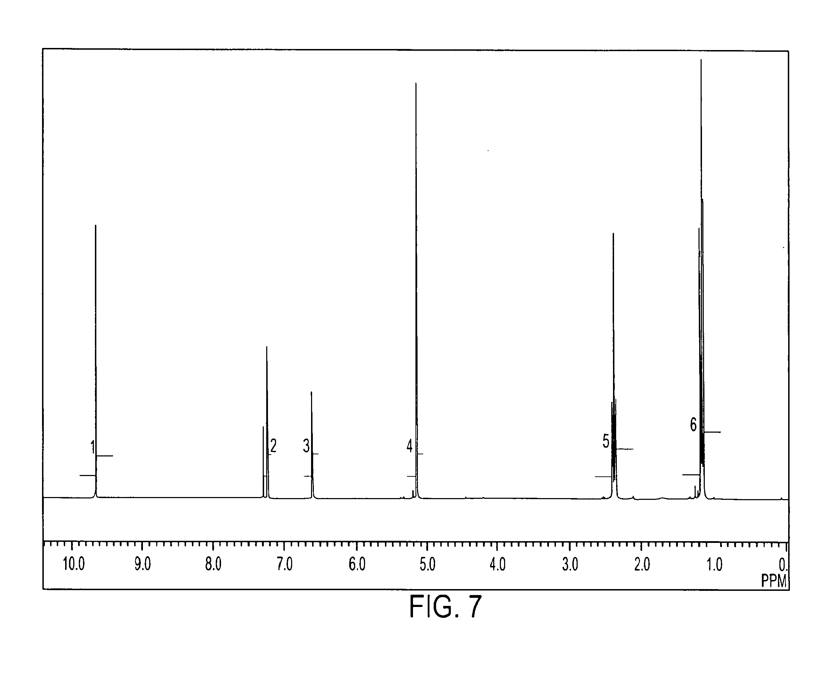

Fig. 7 is a ¹H NMR analysis graph showing substantially pure 5-propionoxymethylfurfual.

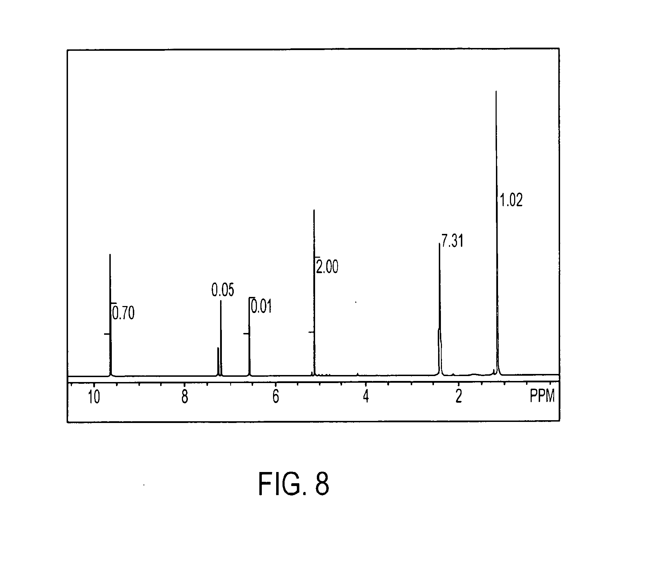

Fig. 8 is a ¹H NMR analysis graph showing substantially pure 2,5-diformylfuran, 2,5-furandicarboxylic acid (FDCA).

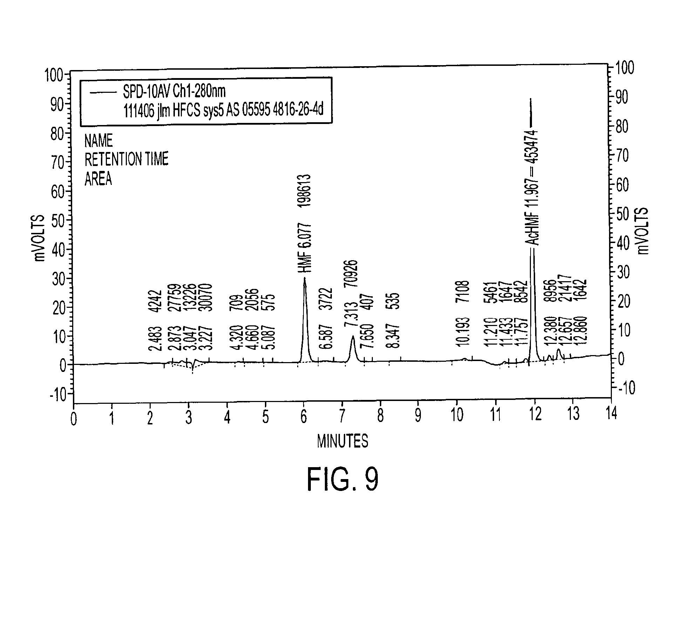

Fig. 9 is a HPLC trace showing the formation of 5-acetoxymethylfurfual (AcHMF) from fructose according to Example 1.

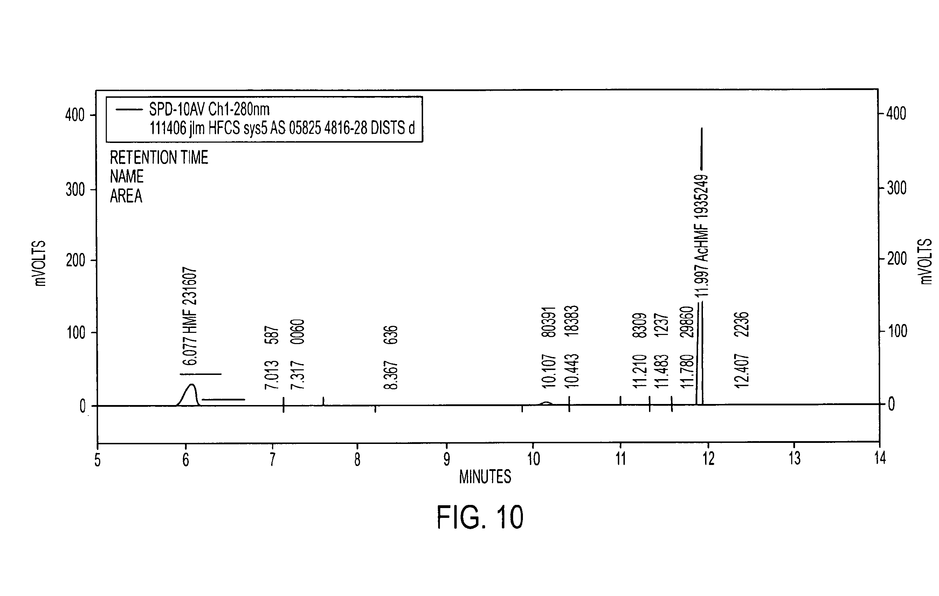

Fig. 10 is a HPLC trace showing the formation of 5-acetoxymethylfurfual (AcHMF) from fructose according to Example 2.

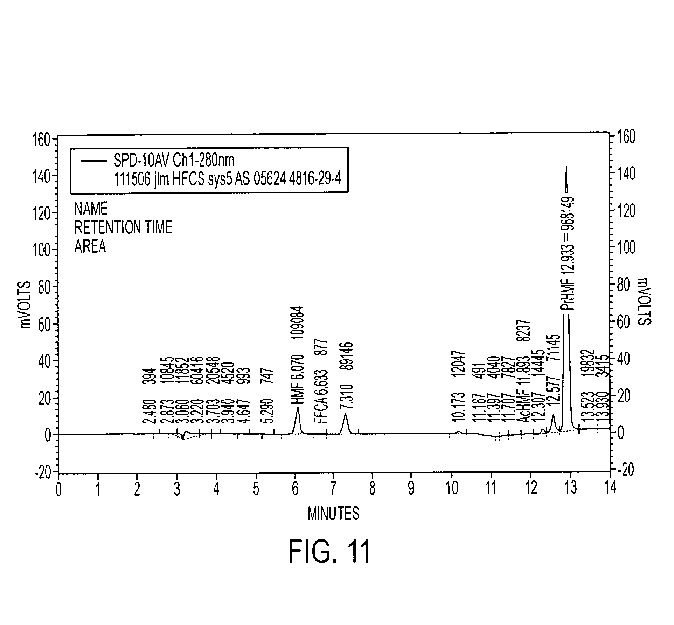

Fig. 11 is a HPLC trace showing the formation of showing substantially pure 5-propionoxymethylfurfual (PrHMF) from fructose.

### DETAILED DESCRIPTION

The present application provides methods for synthesizing and separating hydroxymethylfurfural (HMF) and hydroxymethylfurfural esters from a carbohydrate source by contacting the carbohydrate with a solid phase catalyst.

The use of solid phase catalysts in a chromatography column to synthesize and purify HMF limits exposure time to heat and acid catalysts and enables synthesis at a lower temperature. Lower temperatures result in reduced energy costs and reduced time for heating and cooling the reaction. Non-limiting examples of solid phase catalysts that may be used in the process include acidic resins such as Amberlyst 35, Amberlyst 15, Amberlyst 36, Amberlyst 70, Amberlyst 131 (Rohm and Haas); Lewatit S2328, Lewatit K2431, Lewatit S2568, Lewatit K2629 (Bayer Company); and Dianion SK104, PK228, RCP160, Relite RAD/F (Mitsubishi Chemical America, Inc.). Other solid phase catalysts such as clays and zeolites such as CBV 3024 and CBV 5534G (Zeolyst International), T-2665, T-4480 (United Catalysis, Inc), LZY 64 (Union Carbide), H-ZSM-5 (PQ Corporation) can also be used. Acidic resins such as Amberlyst 35 are cationic, while catalysts such as zeolite, alumina, and clay are porous particles that trap small molecules. Soluble catalysts including inorganic acids, such as H₂SO₄, H₃PO₄, HCl, and organic acids such as p-toluene sulfonic acid may also be used.

An advantage of solid phase catalysts is that they do not dissolve in solvent and remain in the column. Depending on the column size and type of solvent used, about 30-50 g of resin is packed into the column. For example, the solvent dimethylformamide (DMF) causes Amberlyst 35 resin to expand in the column, and thus only about 30 g of resin is preferably used in a 300 mm length column. Approximately 50 g of Amberlyst 35 resin is used when acetic acid is the solvent because acetic acid does not cause the resin to swell.

Because the synthesis of HMF is a dehydration reaction, a cation exchange resin having reduced water content is preferred. The presence of water in the reaction increases formation of byproducts, such as, polymers and humin. Therefore, the maximum water content of the solid phase catalyst in a column experiment is typically less than about 20%, optionally less than about 15%, or less than about 10%. Many commercially available solid phase catalysts, such as, dry Amberlyst 35 have approximately 3% water content. However, solid phase catalysts with greater than 20% may be used under certain conditions. Solid phase catalysts having a water content greater than about 20% are considered "wet resins" due to their excess water content and ability to generate water during the reaction. If the water content of the wet resin is greater than about 20%, a solvent that is miscible with water may be selected as the solvent for the reaction in order to remove water from the wet resin.

Solvents including aprotic polar solvents are preferred because they are miscible with water, which helps with the solubility of fructose and with removing water. An example of an polar aprotic solvent is acetone, which is used to wash the wet resin and dehydrate the wet resin before the reaction on the column. The resulting dehydrated resin is then dried under a vacuum prior to the reaction on the column. In addition, DMF is miscible with water and may be used as a solvent to dehydrate the wet resin on the column. The dehydration of the wet resin may include raising the temperature of the reaction or any suitable method for dehydrating the wet resin or a combination thereof.

An additional advantage of using a column in the conversion of a carbohydrate source to HMF, HMF esters or other HMF derivatives is the ability for the reaction to proceed and separate the product from the unreacted starting material or other unwanted side products that may form all in one step. As the reactants pass through the column, differences in the retention of the products from the starting materials will allow for these to separate after the reaction occurs in the column. As a result, the product will elute from the column in a substantially pure form.

Any carbohydrate source can be used, although fructose is the preferred source. Suitable carbohydrate sources that can be used for preparing HMF derivatives include, but are not limited to, hexose, fructose syrup, crystalline fructose, and process streams from the crystallization of fructose. Suitable mixed carbohydrate sources may comprise any industrially convenient carbohydrate source, such as corn syrup. Other mixed carbohydrate sources include, but are not limited to, hexoses, fructose syrup, crystalline fructose, high fructose corn syrup, crude fructose, purified fructose, high fructose corn syrup refinery intermediates and by-products, process streams from crystallizing fructose or glucose or xylose, and molasses, such as soy molasses resulting from production of soy protein concentrate, or a mixture thereof.

Synthesis of HMF esters from a carbohydrate source and organic acids or acid salts provides a direct pathway for a series of useful molecules. Aliphatic and aromatic esters of HMF are commercially available and have a variety of uses. The present process has many advantages in the production of HMF esters. Suitable carbohydrate sources that can be used for preparing HMF esters include, but are not limited to hexose, fructose syrup, crystalline fructose, and process streams from the crystallization of fructose. Suitable mixed carbohydrate sources may comprise any industrially convenient carbohydrate sources, such as corn syrup. The mixed carbohydrate sources include, but are not limited to, hexoses, fructose syrup, crystalline fructose, high fructose corn syrup, crude fructose, purified fructose, high fructose corn syrup refinery intermediates and by-products, process streams from crystallizing fructose or glucose or xylose, and molasses, such as soy molasses resulting from production of soy protein concentrate. In addition to the wide variety of starting sources, the process can be performed with various organic acids including, but not limited to acetic, propionic, butyric, citric or diacids.

The disclosed process minimizes and/or eliminates the formation of humins and polymeric by-products. If the reaction is not complete and HMF and or unreacted carbohydrate is observed in the reaction mixture, these components may be separated into the aqueous phase and recycled. In addition, the solvents can be recovered and recycled. This method is more beneficial than other methods as it eliminates the difficult task of isolating substantially pure HMF for use as a starting source. It is a simple process, leading to a substantially pure product, which can be used as a feeding source in the transformation of HMF esters to a variety of useful derivatives and end products. The purity of the product will vary according to the particular reagents and conditions used.

Additionally, the present application provides methods for synthesizing citrate esters, and methods of synthesizing levulinic acid or levulinic esters using a heated solid phase catalyst in a column, and subsequent purification of the resulting products in a column. In an example of levulinate ester synthesis, a carbohydrate mixture in solution (e.g. 25% fructose in Acetic acid) is passed through a heated column that is packed with a strong acid cationic resin (e.g. Amberlyst 35). The temperature of the column is maintained at 75C and the flow rate is set to 5mL/min. Upon the initial pass both Ac-HMF and Ac-levulinate acid are formed. Subsequent passes generate a higher ratio of Ac-levulinate to Ac-HMF. The concentration of acetic acid may range from >99% reagent grade acetic acid to 1% acetic acid in aqueous solution.

For the synthesis of a levulinic acid, an example synthesis involves a carbohydrate mixture in solution (e.g. 25% fructose in aqueous or DMF solution) is passed through a heated column that is packed with a strong acid cationic resin (e.g. Amberlyst 35). The temperature of the column is maintained at 100 °C and the flow rate is set to 5mL/min. Upon the initial pass both HMF and levulinic acid are formed. Subsequent passes generate a higher ratio of levulinic acid to HMF.

Various HMF esters are selectively prepared by modifying the choice of solvent used in the processes of the invention. The amount of purification and fractionation of the end product depends on the type of solvent used. For example, a continuous flow of a solution of fructose dissolved in acetic acid through a solid phase catalyst results in the formation of substantially pure acetylated HMF (AcHMF), which is a desired end product. HMF ethers and/or levulinate esters, which are more stable than HMF may be synthesized and purified by this process using an alcohol solvent.

AcHMF has a lower boiling point than HMF, and is isolated by vacuum distillation. AcHMF is also more stable than HMF. AcHMF is not appreciably soluble in water making extraction in a nonpolar organic solvents an effective method of purification. AcHMF crystallizes in nonpolar solvents at low temperatures (e.g., hexanes around 0-25°C). Moreover, HMF decomposes upon heating and produces by-products that are not easily isolated or removed.

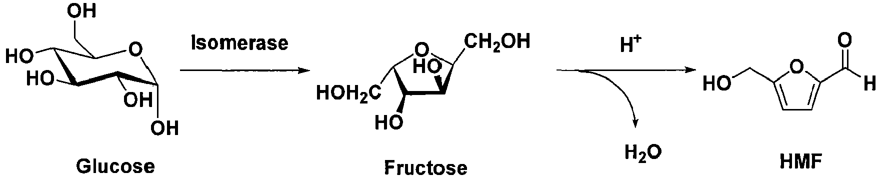

### Synthesis of HMF from glucose and fructose

For one embodiment of the present disclosure, the set up of the chromatography column including a column packed with solid phase catalysts may be a continuous separation where the fructose, HMF, and solvent are fed through the packed column multiple times and/or the speed of additional reactants is varied. This purification technique can include Simulated Moving Bed chromatography, which is a chromatographic technique based on a flow of liquid (mobile phase) moving countercurrent to a constant flow of solid (stationary phase). Countercurrent flow enhances the potential for separation and, hence, makes the process more efficient. It also allows a continuous flow of feed material to be separated, and utilizes less solvent and improves the throughput of the equipment compared to traditional batch chromatography. Alternatively, the system may include, but is not limited to, a simulated moving bed, continuous set up (CSEP), or a continuous flow pipe system.

For example, in Simulated Moving Bed chromatography, the solutes move faster than the bed and are eluted at the top of the column, whereas those moving slower than the bed are brought down by the moving bed below the feed point. A section of the bed below the feed point is then heated to increase the elution rate of the solutes and any solute moving faster than the bed can be eluted through a side tube by a second flow of gas while those solutes still moving at a slower rate than the bed continue to move down the column in the stationary phase. The higher fractions can be removed in the same way by a section of the column heated to an even higher temperature. In order to heat the column in Simulated Moving Bed chromatography, a jacketed column allows the mixture to pass heating fluid, such as, propylene glycol, around the resin bed.

In most of the reactions carried out by the methods described herein, the catalyst provides the necessary acidity for the reaction to occur. Ion exchange resins are synthetic polymers capable of combining or exchanging ions in a surrounding solution and are used primarily for chromatography of organic molecules. Advantages of ion exchange resins include long lifetime, reusable, high selectivity and catalytic ability, stability, and can be used in both aqueous and non-aqueous conditions (Rohm and Haas).

A first type of column that may be used in the disclosed methods is a gravity flow column. The reaction mixture is loaded onto the top of the column, which is heated by a jacket, then allowed to slowly flow through the resin, allowing maximum retention time on the column. The flow rate in a gravity flow column is generally less than 1.0 mL/min, or typically 0.1 - 1.0 mL/min. Once the product is fed through the column, it may be reloaded for a second pass, to produce a higher yield of the desired product and increased purity by allowing more time on the resin. The samples of the gravity column are collected in a large fraction or multiple fractions and analyzed for yield.

Another column that may be used is a pulse column. The starting material is loaded on top of the resin and a mechanical pump is used to pump solvent onto the column to maintain a constant flow rate. The product is collected from the bottom of the column in timed fractions, and, therefore, may be analyzed for retention time, separation of products and reactants, as well as total yield.

For the column experiments, depending on the type of column used and the stability of the solvent, the temperature may be varied from about 70°C to about 125°C, optionally from about 75°C to about 95°C, or optionally from about 80° C to about 90°C. The flow rate is typically kept at about 1.0 ml/minute to allow maximum retention time on the column and flow through to the top of the column. However, for gravity columns, the flow rate may be kept lower, since it is not dependent on a mechanical source. Higher temperatures may be used.

In one embodiment of the present application, Amberlyst 35 is packed in a heated glass jacketed column with fructose solubilized with acetic acid. The use of a continuous moving bed minimizes exposure time of the product to resin since it passes through the column by stream, rather than batch. The resulting product is highly purified acetyl HMF.

In another embodiment of the invention, a continuous flow process enables the formation of citrate esters. The starting material, citric acid may be in solution with the solvent or in a fermentation broth containing solvent. An alcohol solvent is used to obtain either substantially pure trialkyl citrate or the mono- and di-esters of citric acid. Factors such as type of solvent, type of resin, time on column, and/or temperature determine whether mono- and di-esters are formed or whether substantially pure trialkyl citrate is formed. If mono- and di-esters are obtained, the mixture can be recycled in the column to generate the substantially pure triester.

Another aspect of this invention allows for the chromatographic synthesis, separation, and purification of an anhydrosugar alcohol from a selected sugar alcohol or monoanhydrosugar alcohol starting material using a solid phase catalyst. More specifically, isosorbide is synthesized, separated, and purified from a sorbitol source and a solid phase catalyst in a chromatography column. The intermediate compound, sorbitan, or mixtures of sorbitan/isosorbide/sorbitol can be recovered and recycled until substantially pure isosorbide is obtained.

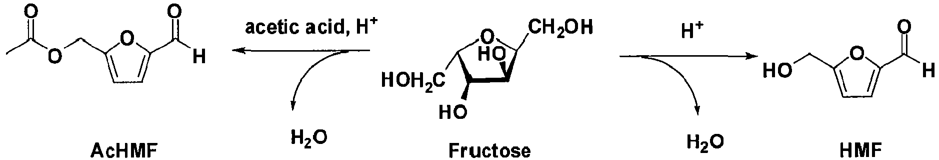

### Synthesis of HMF from AcHMF and Fructose

In another aspect of the invention, there is provided a process for preparing HMF via an intermediate HMF ester. Fructose is preferably used as a carbohydrate source and is subjected to dehydration in the presence of an organic acid and catalyst in a chromatography column to provide a HMF ester. The HMF ester is then subjected to deacylation upon treatment with a base. The basic material may include, but is not limited to, a solid phase catalyst such as resins, clays, aluminas, and zeolites. The saponification of the ester can be carried out in any manner, so long as it can bring the starting material and basic catalyst are brought into mutual contact. For example, this reaction can be carried out batchwise or continuously in a fluidized bed, tubular reactor, coil, column, or pipe. Deacylation of AcHMF to HMF can also be achieved using a metal alkoxide. This method may be preferable due to the fact that HMF esters are generally more stable than HMF.

In another embodiment of forming a HMF ester, a carbohydrate is combined with an organic acid and solid phase catalyst in solution. The solution is heated to a temperature between about 100 to about 140°C, for between about 90 to about 150 minutes, resulting in the formation of an HMF ester. The HMF ester can be further purified via column chromatography, precipitation and recrystallization or combinations thereof. The synthesized HMF ester is filtered and/or evaporated, e.g.,. via rotary evaporation to remove the catalyst and organic acid. HMF ester can be collected by extraction with methyl t-butyl ether. The crude material can be subjected to simple distillation (115°C, 3 torr) to provide the HMF ester as crystals. Alternatively, the filtered HMF ester may then be extracted with a suitable solvent, such as hot hexane, after the evaporation of the solvent.

In one embodiment for the forming of FDCA, a reaction mixture containing a HMF ester such as, but not limited to, 5-acetoxymethylfurfural (AcHMF), or organic acid such as, but not limited to, acetic acid, along with cobalt acetate, manganese acetate and sodium bromide is placed in a reactor and subjected to between about 400 to about 1000 psi, or between about 500 to about 800 psi oxygen at between about 85 °C to about 110 °C, or between at about 100°C for between about 100 to about 150 minutes. The solution is then filtered and the solvent evaporated to obtain 2,5-Furandicarboxylic acid (FDCA).

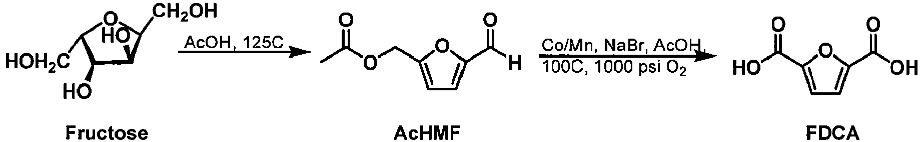

In an embodiment for reducing an HMF ester, a reaction mixture containing AcHMF and ethanol is charged in a reaction vessel. The G-69B catalyst obtained from Sud Chemie, Louisville, KY is added to the vessel and the vessel is purged with hydrogen, preferably at 4X500 psi with stirring, preferably at 1000 rpm. The vessel is then pressurized, preferably to 600 psi and heated to a temperature above 150°C, preferably to 170°C with continual stirring. After about an hour the reaction is heated to 195°C for about an hour and then allowed to cool to room temperature. The catalyst is then removed via vacuum filtration. The solvent is preferably removed, for example, by rotary evaporation. Products of reduction include but are not limited to 2,5-furandimethanol (FDM) and tetrahydrofuran diol (THF-diol).

As an example of the versatility of the HMF-esters, a reaction mixture containing a combination of HMF and an HMF-ester can be oxidized to the single product, FDCA. 2,5-Furandicarboxylic acid (FDCA) is formed from a mixture of predominantly HMF ester with residual HMF in an organic acid. The mixture is reacted in an organic acid, for example acetic acid along with cobalt acetate, manganese acetate and sodium bromide. The entire mixture can be pressurized with oxygen or air and heated to at least 100°C for over an hour. The resulting solution is filtered and evaporated and the FDCA is isolated.

HMF can be synthesized from a fructose source through and HMF ester intermediate. For example, fructose is subjected to dehydration in the presence of an organic acid and catalyst to provide an HMF ester. The HMF ester is subjected to deacylation upon treatment with a base. The basic material may include, but is not limited to, a solid phase catalyst such as resins, clays, aluminas, and zeolites. The saponification of the ester can be carried out in any manner, so long as it can bring the material and basic catalyst into mutual contact. For example, this reaction can be carried out batchwise or continuously in a fluidized bed, tubular reactor, coil, column, or pipe. Deacylation of AcHMF to HMF can also be achieved using a metal alkoxide.

### EXAMPLES

### EXAMPLE 1

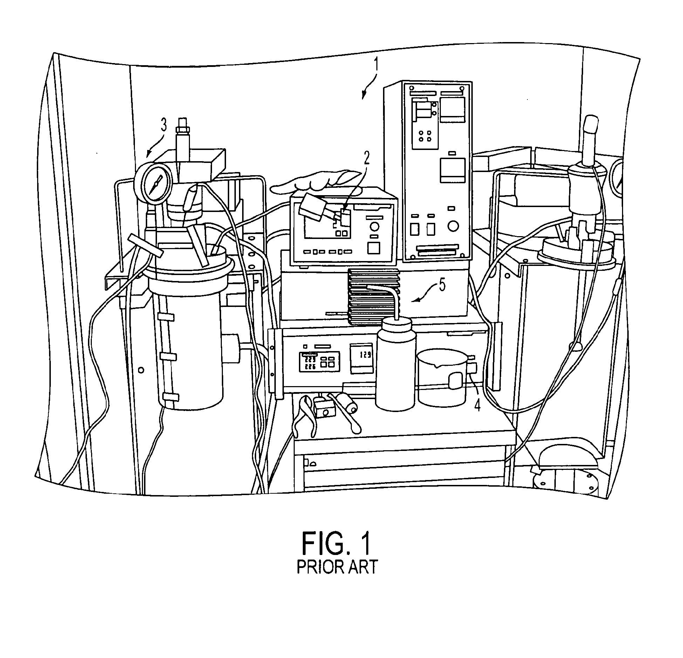

The conventional method for synthesizing HMF and AcHMF from fructose includes a batch reaction on an autoclave (Parr) reactor followed by a separate step for purification. As shown in Fig. 1, the temperature control 2 controls both the temperature of the reaction mixture and the heating jacket in the autoclave reactor 1. A heating jacket (not shown) is used to heat the reaction. The pressure gauge 3 shows if the reaction is creating gas, or monitors the pressure on the vessel if it was applied. The speed control 4 is for the stirring mechanism. Stirring is necessary to keep the reaction mixture in contact with all necessary materials. The sample port 5 allows the scientist to retrieve samples and specific points during the reaction to monitor for progress. Reactants must be in solution before being put into a reactor vessel.

The reaction conditions for the autoclave reactions were varied to test the effect of different reaction conditions. The reactions were performed in the 100 mL capacity Parr reactor. About 20 grams of High fructose corn syrup (HFCS) is added to each reaction. Three different temperatures: 110°, 125°, and 150° Celsius were tested with and without an ion exchange resin. The resin of choice was Amberlyst 35 exchange. Results are shown in Table 1.

**Table 1**

| **Comparative Example** | **#1** | **#2** | **#3** | **#4** | **#5** |
|---|---|---|---|---|---|
| **Temperature** (°C) | 110 | 125 | 125 | 150 | 150 |
| **Resin** | yes | no | Yes | yes | No |
| **Fructose added** (g) | 5.6998 | 5.7995 | 6.7799 | 6.7799 | 6.7799 |
| Moles | .03164 | 0.0322 | 0.0376 | 0.0376 | 0.0376 |
| **Fructose out** (g) | 0.62 | 0.64 | 1.34 | 0.55 | 2.07 |
| Moles | 0.0034 | 0.00354 | 0.0074 | 0.0030 | 0.0115 |
| **HMF out** (g) | 2.13 | 1.22 | 1.60 | 1.48 | 0.79 |
| Moles | 0.0169 | 0.0097 | 0.0127 | 0.0118 | 0.0063 |
| **AcHMF out** (g) | 0.18 | 0.54 | 0.49 | 1.48 | 0.13 |
| Moles | 0.001053 | 0.0033 | 0.0029 | 0.0088 | 0.0007 |
| **HFCS added** (g) | 20 | 20.35 | 23.79 | 23.79 | 23.79 |
| **Fructose added** (g) | 5.6998 | 5.7995 | 6.7799 | 6.7799 | 6.7800 |
| **AcHMF yield** | 0.0332 | 0.1000 | 0.0764 | 0.2331 | 0.0197 |
| **HMF yield** | 0.5348 | 0.3005 | 0.3378 | 0.3125 | 0.1663 |
| **Fructose yield** | 0.1080 | 0.1101 | 0.1974 | 0.0812 | 0.3048 |

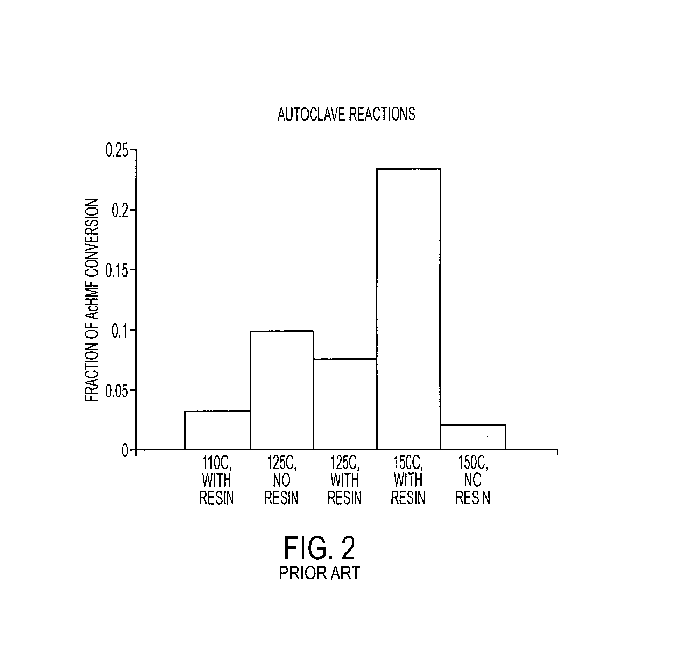

As can be seen in Table 1 above, AcHMF was formed in the largest amount in Comparative Example #4 at 150°C using a resin in an autoclave reactor as shown in Fig. 2. In a 100 mL capacity Parr reactor, 6.7799 g of fructose and 23.79 g of HFCS in solution was heated to 150°C. The AcHMF yield was 0.2331 in Comparative Example #4.

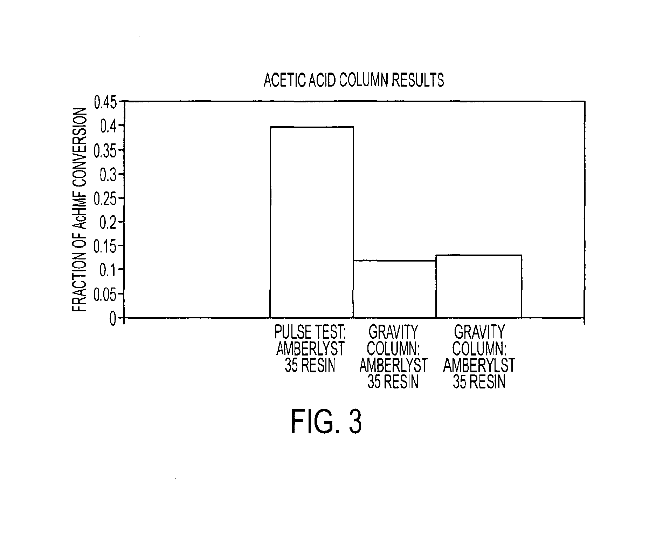

In these examples of Example 1, a first method of production of substantially pure HMF uses packed columns. Two different types of columns were used to produce and purify HMF. Each column, however, was packed with a cation exchange resin, which had been soaked in the desired solvent, then loaded to a heated column once the resin had appropriately expanded. A cation exchange resin is an ion exchange resin that adds protons to the reaction. The water content of the resin used ranged from less than about 20%, to less than about 10% in order to prevent the rehydration of HMF. The results of the columns are shown in Fig. 3. The major product was HMF with the remainder being unreacted fructose. In this example, the Amberlyst 35 exchange resin performed best of all columns tested, including the gravity flow columns.

Maximal reaction conditions included 80° C in a column packed with the Amberlyst 35 ion exchange resin and acetic acid, providing a yield of AcHMF is approximately 0.395 moles. Columns performed more consistently than the conventional batch reactions, which may be due to a number of reasons. Product stays longer on the resin in a chromatography column, and a larger amount of the resin remains in the column than in the batch reactions. There is also better control of the temperature in the column due to the heated jacketed column.

Samples marked with (*) in Table 2 are comparative examples. The comparative examples include batch reactions. The temperatures in the autoclave varied from approximately 105°C - 155° C in the course of the reaction. The reaction mixture from the columns could also be fed back through for another pass, which will further increase the yield of the desired product. The yield is lower during a batch reaction when it is run at a higher temperature, such as 125° C, compared to a pulse reaction at 80° C and a gravity column reaction at 90° C. The yield in the batch reaction using Amberlyst 35 having a temperature of 150° C is increased due to the high temperature.

**Table 2**

| **Type of Reaction** | **Temperature (°C)** | **Type of Resin (if used)** | **AcHMF yield** |
|---|---|---|---|
| Pulse | 80 | Amberlyst 35 | 0.394914426 |
| Gravity Column | 90 | Amberlyst 35 | 0.117696633 |
| Gravity Column | 90 | Amberlyst 35 | 0.130347712 |
| Batch Reaction* | 110 | Amberlyst 35 | 0.033283962 |
| Batch Reaction* | 125 | No resin | 0.100020489 |
| Batch Reaction* | 125 | Amberlyst 35 | 0.076392506 |
| Batch Reaction* | 150 | Amberlyst 35 | 0.233076043 |
| Batch Reaction* | 150 | No resin | 0.019697 |

### EXAMPLE 2

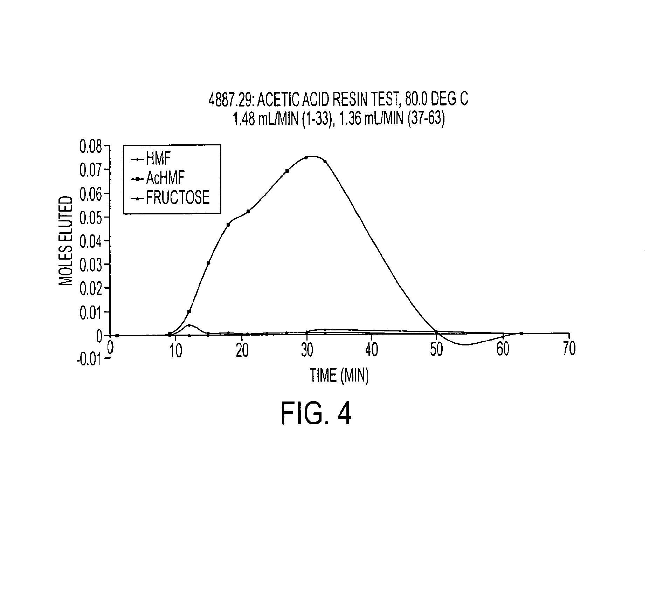

The graph shown in Fig. 4 illustrates the results of the pulse resin test at 80°C where the flow rate was set at about 1.48 mL/min. for the first 33 minutes and about 1.36 mL/min. after the 33rd minute until completion of the reaction at about 63 minutes. After approximately 30 minutes, 0.07 moles of AcHMF was eluted, compared to a mole fraction of approximately 0.0006 for the starting material, fructose. The byproducts, levulinic and formic acids, are also measured. No measurable levulinic acid was found during the synthesis of AcHMF.

**Table 3**

| **Time (min.)** | **Sample weight (g)** | **Percent water in sample** | **Fraction of water in sample** | **Weight of water in sample (g)** | **moles of water in sample** |
|---|---|---|---|---|---|
| 1 | 0.5060 | 2.28 | 0.0228 | 0.0115368 | 0.000640364 |
| 9 | 0.5189 | 1.39 | 0.0139 | 0.00721271 | 0.00040035 |
| 12 | 0.5059 | 1.44 | 0.0144 | 0.00728496 | 0.000404361 |
| 15 | 0.4982 | 1.30 | 0.0130 | 0.0064766 | 0.000359492 |
| 18 | 0.5071 | 1.26 | 0.0126 | 0.00638946 | 0.000354655 |
| 21 | 0.5062 | 1.24 | 0.0124 | 0.00627688 | 0.000348406 |
| 24 | 0.4122 | 1.40 | 0.0140 | 0.0057708 | 0.000320315 |
| 27 | 0.4782 | 1.46 | 0.0146 | 0.00698172 | 0.000387529 |
| 30 | 0.5051 | 1.37 | 0.0137 | 0.00691987 | 0.000384096 |
| 33 | 0.5005 | 1.34 | 0.0134 | 0.0067067 | 0.000372264 |
| 50 | 0.5065 | 1.16 | 0.0116 | 0.0058754 | 0.000326121 |
| 63 | 0.5105 | 1.95 | 0.0195 | 0.00995475 | 0.000552551 |
| | | | | | |

| **Time (min.)** | **Sample weight (g)** | **HMF in sample (g/kg)** | **Fraction of HMF in sample** | **Weight of HMF in sample (g)** | **moles of HMF in sample** |
|---|---|---|---|---|---|
| 1 | 0.5060 | 0.00 | 0.00000 | 0 | 0 |
| 9 | 0.5189 | 0.00 | 0.00000 | 0 | 0 |
| 12 | 0.5059 | 1.49 | 0.00149 | 0.000752273 | 5.96514E-06 |
| 15 | 0.4982 | 0.23 | 0.00023 | 0.000116081 | 9.20459E-07 |
| 18 | 0.5071 | 0.28 | 0.00028 | 0.000143002 | 1.13393E-06 |
| 21 | 0.5062 | 0.12 | 0.00012 | 5.87192E-05 | 4.65613E-07 |
| 24 | 0.4122 | 0.27 | 0.00027 | 0.000109233 | 8.66161E-07 |
| 27 | 0.4782 | 0.25 | 0.00025 | 0.000117159 | 9.2901E-07 |
| 30 | 0.5051 | 0.28 | 0.00028 | 0.000141933 | 1.12546E-06 |
| 33 | 0.5005 | 0.36 | 0.00036 | 0.000181682 | 1.44064E-06 |
| 50 | 0.5065 | 0.00 | 0.00000 | 0 | 0 |
| 63 | 0.5105 | 0.00 | 0.00000 | 0 | 0 |
| | | | | | |

| **Time (min.)** | **Sample weight (g)** | **Acetic Acid in sample (g/kg)** | **Fraction of Acetic Acid in sample** | **Weight of Acetic Acid in sample (g)** | **moles of Acetic Acid in sample** |
|---|---|---|---|---|---|
| 1 | 0.5060 | 960.17 | 0.9602 | 0.48584349 | 0.008090326 |
| 9 | 0.5189 | 967.75 | 0.9678 | 0.502167551 | 0.008362156 |
| 12 | 0.5059 | 896.88 | 0.8969 | 0.453733616 | 0.007555628 |
| 15 | 0.4982 | 924.96 | 0.9250 | 0.460815072 | 0.00767355 |
| 18 | 0.5071 | 907.85 | 0.9078 | 0.460369214 | 0.007666125 |
| 21 | 0.5062 | 846.98 | 0.8470 | 0.428742288 | 0.00713947 |
| 24 | 0.4122 | 871.93 | 0.8719 | 0.359409958 | 0.005984939 |
| 27 | 0.4782 | 880.87 | 0.8809 | 0.421230121 | 0.007014376 |
| 30 | 0.5051 | 876.47 | 0.8765 | 0.442705502 | 0.007371987 |
| 33 | 0.5005 | 857.03 | 0.8570 | 0.428941013 | 0.007142779 |
| 50 | 0.5065 | 903.94 | 0.9039 | 0.45784713 | 0.007624127 |
| 63 | 0.5105 | 980.54 | 0.9805 | 0.50056567 | 0.008335482 |
| | | | | | |

| **Time (min.)** | **Sample weight (g)** | **Fructose in sample (g/kg)** | **Fraction of Fructose in sample** | **Weight of Fructose in sample (g)** | **moles of Fructose in sample** |
|---|---|---|---|---|---|
| 1 | 0.5060 | 0.00 | 0.0000 | 0 | 0 |
| 9 | 0.5189 | 0.04 | 0.0000 | 1.91993E-05 | 1.0657E-07 |
| 12 | 0.5059 | 0.17 | 0.0002 | 8.54971E-05 | 4.74569E-07 |
| 15 | 0.4982 | 0.12 | 0.0001 | 6.02822E-05 | 3.34609E-07 |
| 18 | 0.5071 | 0.00 | 0.0000 | 1.5213E-06 | 8.44429E-09 |
| 21 | 0.5062 | 0.18 | 0.0002 | 9.26346E-05 | 5.14188E-07 |
| 24 | 0.4122 | 0.45 | 0.0004 | 0.000184666 | 1.02502E-06 |
| 27 | 0.4782 | 0.28 | 0.0003 | 0.000132461 | 7.35255E-07 |
| 30 | 0.5051 | 0.64 | 0.0006 | 0.000323264 | 1.79434E-06 |
| 33 | 0.5005 | 1.03 | 0.0010 | 0.000513513 | 2.85036E-06 |
| 50 | 0.5065 | 0.30 | 0.0003 | 0.00015195 | 8.4343E-07 |
| 63 | 0.5105 | 0.00 | 0.0000 | 0 | 0 |

| **Time (min.)** | **Sample weight (g)** | **Formic in sample (g/kg)** | **Fraction of Formic in sample** | **Weight of Formic in sample (g)** | **moles of Formic in sample** |
|---|---|---|---|---|---|
| 1 | 0.5060 | 0.69 | 0.0007 | 0.000351164 | 7.62975E-06 |
| 9 | 0.5189 | 0.64 | 0.0006 | 0.000331577 | 7.20419E-06 |
| 12 | 0.5059 | 0.75 | 0.0007 | 0.000377401 | 8.19981E-06 |
| 15 | 0.4982 | 1.28 | 0.0013 | 0.0006367 | 1.38336E-05 |
| 18 | 0.5071 | 4.05 | 0.0040 | 0.00205122 | 4.45669E-05 |
| 21 | 0.5062 | 2.56 | 0.0026 | 0.001296884 | 2.81775E-05 |
| 24 | 0.4122 | 3.16 | 0.0032 | 0.001301728 | 2.82827E-05 |
| 27 | 0.4782 | 3.39 | 0.0034 | 0.001621098 | 3.52217E-05 |
| 30 | 0.5051 | 6.29 | 0.0063 | 0.003175564 | 6.89956E-05 |
| 33 | 0.5005 | 6.45 | 0.0065 | 0.003229727 | 7.01724E-05 |
| 50 | 0.5065 | 1.74 | 0.0017 | 0.00088283 | 1.91813E-05 |
| 63 | 0.5105 | 0.70 | 0.0007 | 0.000354798 | 7.7087E-06 |
| | | | | | |

| **Time (min.)** | **Sample weight (g)** | **Levulinic in sample (g/kg)** | **Fraction of Levulinic in sample** | **Weight of Levulinic in sample (g)** | **moles of Levulinic in sample** |
|---|---|---|---|---|---|
| 1 | 0.5060 | 0.00 | 0.0000000 | 0 | 0 |
| 9 | 0.5189 | 0.00 | 0.0000000 | 0 | 0 |
| 12 | 0.5059 | 0.00 | 0.0000000 | 0 | 0 |
| 15 | 0.4982 | 0.00 | 0.0000000 | 0 | 0 |
| 18 | 0.5071 | 0.00 | 0.0000000 | 0 | 0 |
| 21 | 0.5062 | 0.00 | 0.0000000 | 0 | 0 |
| 24 | 0.4122 | 0.00 | 0.0000000 | 0 | 0 |
| 27 | 0.4782 | 0.00 | 0.0000000 | 0 | 0 |
| 30 | 0.5051 | 0.00 | 0.0000000 | 0 | 0 |
| 33 | 0.5005 | 0.00 | 0.0000000 | 0 | 0 |
| 50 | 0.5065 | 0.00 | 0.0000000 | 0 | 0 |
| 63 | 0.5105 | 0.00 | 0.0000000 | 0 | 0 |
| | | | | | |

| **Time (min.)** | **Sample weight (g)** | **AcHMF in sample (g/kg)** | **Fraction of AcHMF in sample** | **Weight of AcHMF in sample (g)** | **moles of AcHMF in sample** |
|---|---|---|---|---|---|
| 1 | 0.5060 | 0.00 | 0.0000000 | 0 | 0 |
| 9 | 0.5189 | 0.22 | 0.0002200 | 0.000114158 | 6.75092E-07 |
| 12 | 0.5059 | 5.01 | 0.0050070 | 0.002533041 | 1.49795E-05 |
| 15 | 0.4982 | 15.21 | 0.0152050 | 0.007575131 | 4.47968E-05 |
| 18 | 0.5071 | 22.98 | 0.0229820 | 0.011654172 | 6.89188E-05 |
| 21 | 0.5062 | 27.97 | 0.0279720 | 0.014159426 | 8.3734E-05 |
| 24 | 0.4122 | 31.95 | 0.0319470 | 0.013168553 | 7.78744E-05 |
| 27 | 0.4782 | 36.23 | 0.0362280 | 0.01732423 | 0.00010245 |
| 30 | 0.5051 | 37.00 | 0.0370010 | 0.018689205 | 0.000110522 |
| 33 | 0.5005 | 36.52 | 0.0365190 | 0.01827776 | 0.000108088 |
| 50 | 0.5065 | 0.35 | 0.0003540 | 0.000179301 | 1.06033E-06 |
| 63 | 0.5105 | 0.00 | 0.0000000 | 0 | 0 |

### EXAMPLE 3

### PREPARATION OF HMF FROM FRUCTOSE USING A HMF ESTER INTERMEDIATE

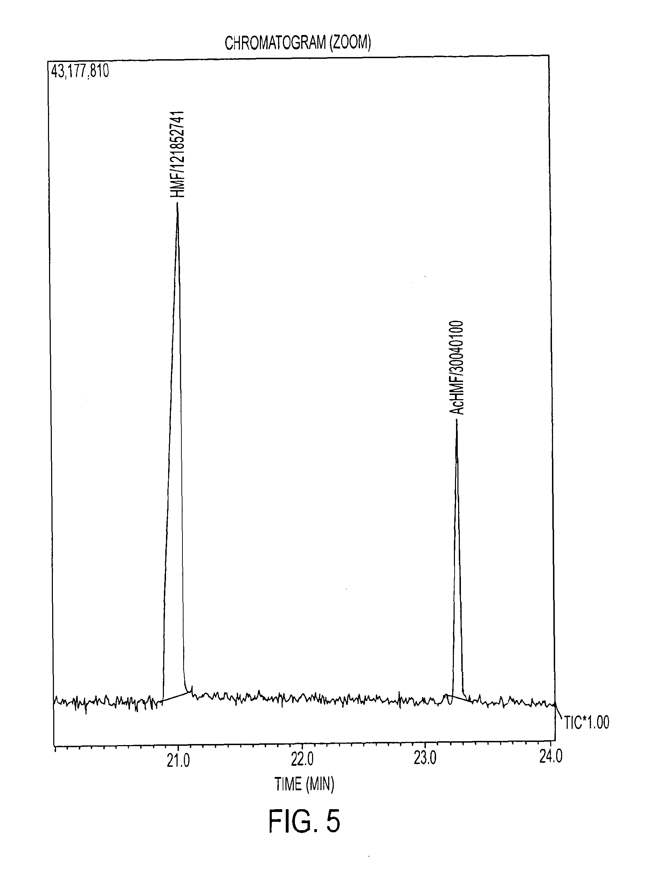

This example illustrates the use of the present methods to deprotect HMF ester to provide substantially pure HMF. The feed material was prepared and placed in a vial of methanol and Amberlyst A26OH resin obtained from Rohm and Haas Company (Woodridge, IL). Amberlyst A26OH resin is a strong base, type 1, anionic, macroreticular polymeric resin based on crosslinked styrene divinylbenzene copolymer containing quaternary ammonium groups. After sitting at room temperature for about 5 minutes, the material was analyzed by thin layer chromatography (tlc) to show deacylation. The solid yield was about 85% HMF with about 8% AcHMF determined by a Shimadzu QP-2010 GC Mass spectrometer. The chromatogram is shown in Fig. 5. The remaining material was residual methanol. Heating the methanol solution with a heat gun to 60 °C for less than 5 minutes converted the remaining AcHMF to HMF. Alternatively, passing the product through the chromatography column with a solid phase catalyst would convert the remaining AcHMF to HMF.

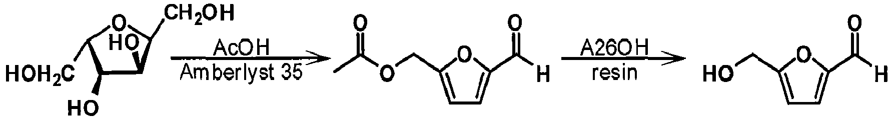

### EXAMPLE 4

### PREPARATION OF 5-ACETOXYMETHYLFURFURAL (AcHMF) FROM FRUCTOSE

Crystalline fructose (18g) is placed in a 100 mL reaction vessel with acetic acid (50 g) and Amberlyst 15 resin (4 g). The solution is heated to 110°C for 3 hours with samples collected regularly. Analytical results and HPLC trace confirm the formation of AcHMF. Analysis of the product mixture indicated a solution of 9.89% AcHMF and 5.14% HMF for a 41% yield of AcHMF and 28% yield of HMF. The yields disclosed herein are exemplary only and do not necessarily reflect the optimal yields possible when reaction conditions are optimized. HPLC trace confirmed formation of AcHMF, (see Fig. 4).

### EXAMPLE 5

### SYNTHESIS AND PURIFICATION OF AcHMF FROM FRUCTOSE

Crystalline fructose (180 g) is placed in a 1 L reaction vessel with acetic acid (500 g) and Amberlyst 15 resin (40 g). The solution is heated to 125 °C for 2 hours. NMR and analytical results indicates the formation of AcHMF. The solution is filtered to remove the resin catalyst and acetic acid is removed by rotary evaporation. AcHMF is collected by extraction with methyl t-butyl ether. The crude material is subjected to simple distillation (115°C, 3 torr) to provide AcHMF as orange crystals. HPLC trace confirms AcHMF formation, (see Fig. 5), and ¹H NMR analysis indicates substantially pure AcHMF. NMR (δ, 1 H): 9.70 (s, 1.0 H); 7.40 (s, 1.0 H); 6.80 (s, 1.0 H); 5.10 (s, 2.0 H); 2.10 (s, 3.0H). See Fig. 1.

### EXAMPLE 6

### SYNTHESIS OF PROPIONOXYMETHYLFURFURAL (PrHMF) FROM FRUCTOSE

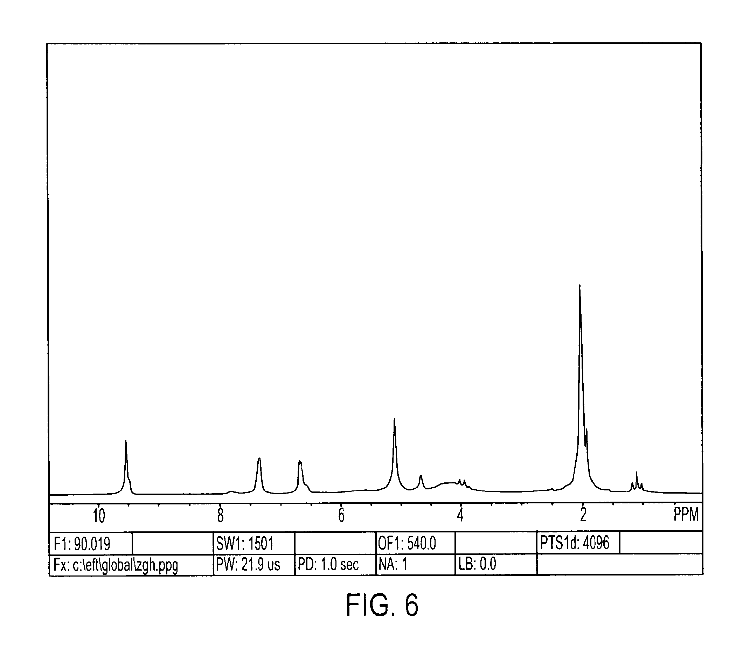

Crystalline fructose (40 g), propionic acid (100 mL), and dry Amberlyst 15 resin is placed in a 500 mL three neck round bottom flask equipped with a Dean-Stark trap, magnetic stir bar, and temperature probe. The reaction mixture is allowed to heat to 130°C for 30 minutes. HPLC trace indicates rapid conversion to the HMF ester. Resin is removed by filtration, the solvent evaporated and the crude oil is extracted with hot hexane. Evaporation of the hexane extract provides yellow oil which ¹H NMR identified as substantially pure 5-propionoxymethylfurfural. Calculations indicate the overall yield of PrHMF from fructose is 28%. Reaction conditions have not been optimized. HPLC trace confirms PrHMF formation , (see Fig. 6) and ¹H NMR analysis indicates PrHMF formation (δ, 1 H): 9.70 (s, 1.0 H); 7.20 (s, 1.0 H); 6.60 (s, 1.0 H); 5.06 (s, 2.0H); 2.47 (t, 2.0 H); 1.05 (d, 3.0H). See Fig. 2.

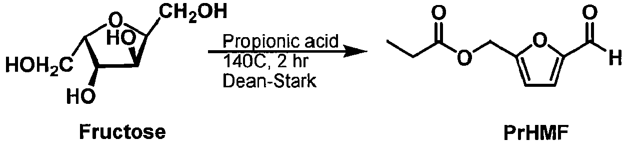

### EXAMPLE 7

### OXIDATION OF 5-ACETOXYMETHYLFURFURAL (AcHMF) TO 2,5-FURANDICARBOXYLIC ACID (FDCA)

A reaction mixture containing AcHMF (5.0 g), acetic acid (50 mL), cobalt acetate (0.132 g), manganese acetate (0.135 g), and sodium bromide (0.114 g) is placed in a 100 mL reactor and subjected to 500-800 psi oxygen at 100°C for 2 hours. Upon filtration and evaporation of the solvent, 2.53 g of tan solid is isolated. ¹H NMR indicates substantially pure FDCA. The overall yield of FDCA from AcHMF is 54%. See Fig. 3.

### EXAMPLE 8

### REDUCTION OF 5-ACETOXYMETHYLFURFURAL (AcHMF)

A reaction mixture containing AcHMF (5.0 g) and ethanol (50 mL) is charged into a 100 mL reaction vessel. The G-69B catalyst may be obtained from Sub Chemie, Louisville, KY (0.50 g) is added to the vessel. The vessel is purged with hydrogen (4X500 psi) with stirring (1000 rpm). The vessel is then pressurized to 600 psi and heated to 170°C with continual stirring. After 1 hour, the reaction is allowed to heat to 195°C for an additional hour. The reaction is then allowed to cool to room temperature and the catalyst removed by vacuum filtration. Most of the solvent is removed by rotary evaporation to provide yellow oil (16.97 g). The UV analysis (λ=284 nm) does not show the presence of AcHMF, indicating complete conversion of AcHMF to 2,5-dihydroxymethyltetrahydrofuran.

### EXAMPLE 9

### OXIDATION OF A MIXTURE OF HMF AND HMF ESTER TO FDCA

A product mixture composed of predominantly HMF ester with residual HMF in acetic acid is subjected to oxidation with the addition of cobalt acetate, manganese acetate, and sodium bromide. This mixture is pressurized with oxygen and heated to over 100°C for over an hour. Upon filtration and evaporation, a product of FDCA is isolated.

### EXAMPLE 10

### PREPARATION OF HMF FROM FRUCTOSE

To a jacketed column was added a slurry of Amberlyst 35 dry resin (40 g) in DMF. The column was heated by an oil circulating bath at 95 °C. The resin was washed with anhydrous DMF. The level of DMF was then lowered to the top of the resin. The column was then loaded with 150 g of 30% fructose in DMF. The fructose solution was passed slowly through the resin over a period of about 1 hour two times. Tlc and NMR analysis indicate 68% yield of HMF.

It will be understood that certain of the above-described structures, functions and operations of the above-described preferred embodiments are not necessary to practice the present disclosure and are included in the description simply for completeness of an exemplary embodiment or embodiments. In addition, it will be understood that specifically described structures, functions, and operations set forth in the above-referenced patents can be practiced in conjunction with the present disclosure, but they are not essential to its practice.

The embodiments of the disclosure may be practiced otherwise than as specifically described without actually departing from the spirit and scope of the present disclosure. The yields disclosed herein are exemplary only and do not necessarily reflect the optimal yields possible when reaction conditions are optimized.
The invention further includes the following embodiments:

### Items

1. A method for oxidising an ester of 2-hydroxymethyl-5-furfuraldehyde (HMF) to 2,5-furandicarboxylic acid (FDCA) by combining the HMF with an organic acid, under pressure in the presence of cobalt, manganese and bromide.
2. The method of item 1 wherein said HMF ester further comprises HMF.
3. The method of item 1 or item 2 wherein the cobalt, manganese and bromide are cobalt acetate, manganese acetate and sodium bromide.
4. A continuous method of item 1 or item 2 of preparing 2,5-furandicarboxylic acid from a hexose carbohydrate starting material, comprising:
   heating a hexose carbohydrate source and solvent in a column;
   continuously flowing the heated carbohydrate source and solvent through a solid phase catalyst in the presence of an organic acid to form a corresponding ester of 2-hydroxymethyl-5-furfuraldehyde; and under pressure, oxidizing the ester to 2,5-furandicarboxylic acid with an organic acid and oxygen or air in the presence of cobalt, manganese and bromide.
5. A method according to item 4, wherein the 2,5-furandicarboxylic acid product is recovered as a substantially pure solid by precipitation out of the solvent, subsequent filtration and evaporation of residual solvent, without requiring any isolation or purification of the ester before it is oxidized to the 2,5-furandicarboxylic acid product.
6. A method according to either of items 4 or 5, wherein the hexose carbohydrate source includes one of more of hexoses, fructose syrup, crystalline fructose, high fructose corn syrup, purified fructose, high fructose corn syrup refinery intermediates and byproducts, process streams from crystallizing fructose or glucose or xylose, and molasses.
7. A method according to item 6, wherein the hexose carbohydrate source is substantially high fructose corn syrup.
8. A method according to any of items 4-7, wherein a solid phase catalyst is provided in the form of a cation exchange resin which is suited for removing impurities and byproducts as these are formed and thereby eluting a substantially pure 2-hydroxymethyl-5-furfuraldehyde ester stream or a substantially pure combined stream of 2-hydroxymethyl-5-furfuraldehyde and 2-hydroxymethyl-5-furfuraldehyde ester for oxidizing to the desired 2,5-furandicarboxylic acid product, said cation exchange resin further being characterized as having a reduced water content of less than 20 percent.
9. A method according to any of items 4-8, wherein the organic acid is selected from formic acid, acetic acid, propionic acid, butyric acid, organic diacids and mixtures of any of these.
10. A method according to any of items 4-9, wherein the formation of the ester occurs at a temperature of between 70 and 125 degrees Celsius, and the ester is then oxidized with pressurized oxygen or air at 400 to 1000 pounds per square inch and a temperature of from 85 to 110 degrees Celsius over the course of an hour or more.
11. A continuous method of item 4 for preparing a substantially pure 2,5-furandicarboxylic acid product from fructose, comprising solubilizing fructose in acetic acid and flowing the solubilized fructose feed in a heated column packed with an acidic, cation exchange resin to provide an acetyl ester of 2-hydroxymethyl-5-furfuraldehyde, and then oxidizing the acetyl ester so formed with oxygen or air in the presence or acetic acid, cobalt acetate, manganese acetate and sodium bromide under elevated pressure and temperature and for a time sufficient to substantially fully convert all of the acetyl ester formed to the desired 2,5-furandicarboxylic acid product.
12. A continuous method of forming one or both of 2,5-furandimethanol and 2,5-tetrahydrofurandiol from a hexose carbohydrate source, comprising:
   heating a hexose carbohydrate source and solvent in a column;
   continuously flowing the heated carbohydrate source and solvent through a solid phase catalyst in the presence of an organic acid to form a corresponding ester of 2-hydroxymethyl-5-furfuraldehyde; and combining the ester with an alcohol in the presence of a catalyst and under elevated pressure and temperature conditions, for a time sufficient to substantially fully convert all of the ester formed to a reduced product stream including one or both of 2,5-furandimethanol and 2,5-tetrahydrofurandiol.

## Claims

1. A method for oxidizing a reaction mixture of 2-hydroxymethyl-5-furfuraldehyde (HMF) and an ester of 2-hydroxymethyl-5-furfuraldehyde (HMF) to 2,5-furandicarboxylic acid (FDCA) by the addition of cobalt acetate, manganese acetate, sodium bromide under pressure and heat to form FDCA.

2. The method according to claim 1, wherein the 2,5-furandicarboxylic acid (FDCA) product is isolated, in particular as a single product, after filtration and evaporation.

3. The method according to claim 1 or 2, wherein the mixture is pressurized with oxygen or air.

4. The method according to any one of claims 1 to 3, wherein the mixture is heated to at least 100 °C for over an hour.

5. The method according to any one of claims 1 to 4, wherein the mixture contains predominantly HMF ester and residual HMF.

6. The method according to any one of claims 1 to 5, wherein the mixture comprises an organic acid.

7. The method according to claim 6, wherein the organic acid is acetic acid.

8. The method according to claim 1, wherein the mixture contains predominantly HMF ester and residual HMF in acetic acid and the mixture is pressurized with oxygen or air and heated to at least 100°C for over an hour and the 2,5-furandicarboxylic acid (FDCA) product is isolated after filtration and evaporation.
